# EUROPEAN PATENT APPLICATION

(11) **EP 0 675 126 A1**
(43) Date of publication of application: **04.10.1995**
(21) Application number: 94930347.3
(22) Date of filing: 20.10.1994
(51) Int. Cl.: C07D 498/18

(54) **AMYLOID BETA-PROTEIN AGGLUTINATION AND/OR DEPOSITION INHIBITOR**

(30) Priority: 20.10.1993 JP 262252/93; 15.03.1994 JP 43976/94; 31.05.1994 JP 118266/94; 02.08.1994 JP 181423/94
(71) Applicant: TEIJIN LIMITED, Osaka-shi, Osaka 541 (JP)
(72) Inventor: TOMIYAMA, Takami Teijin Limited Tokyo Research, Hino-shi Tokyo 191 (JP); SUWA, Yorimasa Teijin Limited, Tokyo Research, Hino-shi Tokyo 191 (JP); ASANO, Satoshi Teijin Limited, Tokyo Research, Hino-shi Tokyo 191 (JP); KATAOKA, Kenichiro Teijin Limited, Tokyo Research, Hino-shi Tokyo 191 (JP); MORITA, Takuya Teijin Limited, Tokyo Research, Hino-shi Tokyo 191 (JP); ENDO, Noriaki Teijin Limited, Tokyo Research, Hino-shi Tokyo 191 (JP)
(74) Representative: Harrison, Ivor Stanley
(86) International application number: JP9401770
(87) International publication number: WO9511248

(57) **Abstract**

An amyloid β-protein agglutination and/or deposition inhibitor containing rifamycin represented by formula (I) or a pharmaceutically acceptable salt thereof as the active ingredient; and a drug containing the same for treating or preventing senile dementia of Alzheimer type.

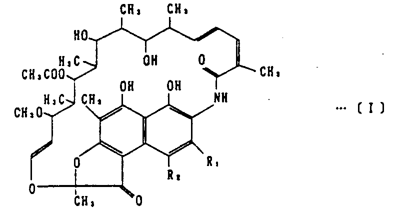

## Description

### TECHNICAL FIELD

The present invention relates to a β-amyloid protein aggregation and/or deposition inhibitor. More specifically, it relates to a β-amyloid protein aggregation and/or deposition inhibitor which contains a rifamycin as an active component.

### BACKGROUND ART

β-amyloid protein is a major component which forms the amyloid core of senile plaque, one of the characteristic pathological changes observed in the brain of Alzheimer's disease patients, and it is a peptide comprising 39-43 amino acid residues which is produced by enzymatic cleavage of the membrane-spanning form of amyloid precursor protein (APP) (See, for example, MORI, H. et al. (1992), The Journal of Biological Chemistry, Vol.267, pp.17082-17086; LANSBURY, P.T., Jr. (1992), Biochemistry, Vol.31, pp.6865-6870; SISODIA, S.S. et al. (1990), Science, Vol.248, pp.492-495; MULLAN, M. et al. (1993), Trends in Neuroscience, Vol.16, pp.398-403).

Based on experiments using chemically synthesized β-amyloid protein, it has been reported that such β-amyloid protein has self-aggregation properties (See, for example, JARRETT, J.T. et al. (1993), Cell, Vol.73, pp.1055-1058; BURDICK, D. et al. (1992), The Journal of Biological Chemistry, Vol.267, pp.546-554; FRASER, P.E. et al. (1992), Biochemistry, Vol.31, pp.10716-10723), and that aggregated β-amyloid protein exhibits direct cytotoxicity against nerve cells and increases cellular sensitivity to injury caused by excitatory amino acids (See, for example, PIKE, C.J. et al. (1993) The Journal of Neuroscience, Vol.13, pp.1676-1687; MATTSON, M.P. et al. (1993), Trends in Neuroscience, Vol.16, pp.409-414).

It has also been reported that a partial peptide of β-amyloid protein, β25-35 peptide (amino acid sequence: GSNKGAIIGLM) exhibits potent neurotoxicity, that one of the action sites of the β25-35 peptide is the mitochondria electron transfer system of nerve cells, and that the effect of β25-35 peptide on cellular redox activity which can be measured using MTT (3-(4,5-dimethylthiazol-2-yl)-2,5-diphenyltetrazolium bromide) may reflect the strength of its cytotoxicity (Yanker, et al., Science, 250, 279-282, 1990; Kaneko, et al. Shinkei Kagaku, 32, 148-149, 1993).

Furthermore, the formation of senile plaque in the brain by deposition of β-amyloid protein is known to be a pathological change which appears at an earlier stage than neurofibrillary tangle, another characteristic pathological change in the brain of Alzheimer's disease patients (See, for example, SEJKOE, D.J. (1991), Neuron, Vol.6, pp.487-498; RUMBLE, B. et al. (1989), The New England Journal of Medicine, Vol.320, pp.1446-1452).

Consequently, in Alzheimer's disease, aggregation and deposition of β-amyloid protein in brain tissue is believed to be the trigger causing formation of senile plaque and provoking neuronal death, and thereby to constitute a strong mechanism for the onset of dementia.

Accordingly, drugs which inhibit the aggregation and/or deposition of β-amyloid protein are expected to be useful as therapeutic or preventive medicines against Alzheimer-type dementia. However, there are no reports on low molecular compounds with such inhibitory activity.

On the other hand, rifamycins, which include antibiotics produced by *Streptomyces mediterranei*, such as rifamycin B, rifamycin S, etc. and semisynthetic antibiotics such as rifamycin SV and rifampicin, are known to exhibit a strong antibacterial effect against tubercule bacilli. It has not been reported that these rifamycins have inhibitory activity against aggregation and/or deposition of β-amyloid protein.

### DISCLOSURE OF THE INVENTION

It is an object of the present invention to provide a drug which inhibits the aggregation and/or deposition of β-amyloid protein. It is also an object of the present invention to provide a therapeutic or preventive medicine against Alzheimer's disease.

As a result of diligent research on the prospects of drugs which inhibit the aggregation and/or deposition of β-amyloid protein, the present inventors have found that the rifamycins according to the present invention have inhibitory activity against the aggregation and/or deposition of β-amyloid protein, as well as activity of suppressing neurotoxicity due to β-amyloid protein, and the present invention has thus been completed.

In other words, the present invention relates to a β-amyloid protein aggregation and/or deposition inhibitor which contains as an active component a rifamycin represented by the following formula [I] 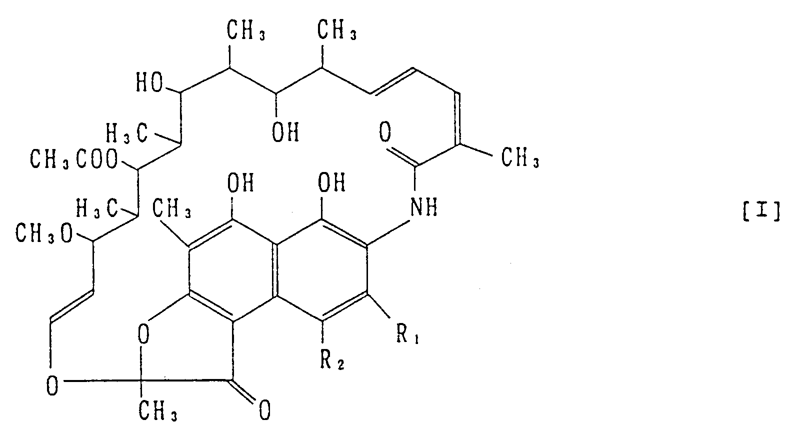
wherein R₁ is a hydrogen atom or the group represented by the following formula [II] 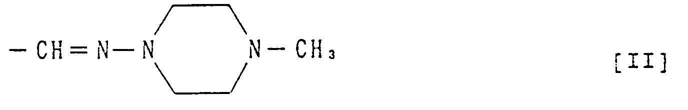
and R₂ is a hydroxyl group or the group represented by the following formula [III] or [IV]

-OCH₂COOH [III]

-OCH₂CON(C₂H₅)₂ [IV]

or R₁ and R₂ may together represent the cyclic structure represented by the following formula [V] 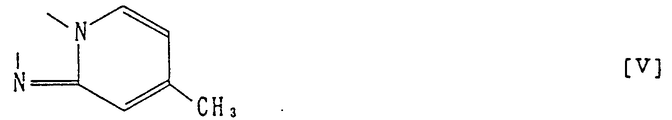
or by the following formula [VI] 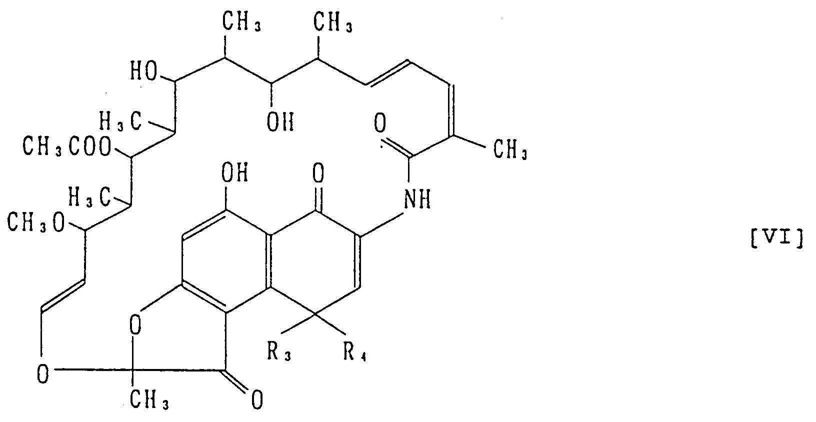
wherein R₃ and R₄ together form either a carbonyl group with the carbon atom to which they are bonded or the group represented by the following formula [VII] 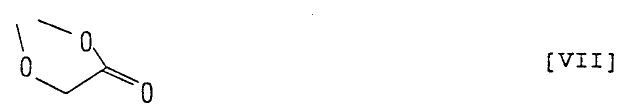
or a pharmaceutically acceptable salt thereof, as well as to a therapeutic or preventive medicine against Alzheimer-type dementia which contains a rifamycin represented by formula [I] or [VI] above as the rifamycin constituting its effective component, or a pharmaceutically acceptable salt thereof, and particularly it relates to a therapeutic or preventive medicine against Alzheimer-type dementia, which is based on the inhibitory effect against aggregation and/or deposition of β-amyloid protein.

### BRIEF DESCRIPTION OF THE DRAWINGS

Fig. 1 shows the suppressing effect of rifampicin on β25-35 peptide cytotoxicity which was measured by the MTT assay using PC12 cells, according to Example 3.

Fig. 2 shows the suppressing effect of rifamycin derivatives of the present invention on β1-35 peptide aggregation, according to Example 4.

In this figure, A to E correspond respectively to rifampicin (A), rifamycin B (B), rifamycin SV (C), rifamycin S (D) and dimethylsulfoxide (E), and the graph bars a to e correspond respectively to day 0, day 10, day 14, day 21 and day 29.

Fig. 3 shows the suppressing effect of rifampicin on β1-40 peptide aggregation, according to Example 5.

In this figure, A to E correspond respectively to no addition (control), 1% dimethylsulfoxide, 100 µg/ml of rifampicin, 10 µg/ml of rifampicin and 1 µg/ml of rifampicin, while the graph bars a to d correspond respectively to day 0, day 1, day 3 and day 6.

Fig. 4 shows the suppressing effect of rifampicin on β1-40 cytotoxicity which was measured by the LDH assay using PC12 cells, according to Example 7.

In this figure, the solid white graph bars indicate cell damage of PC12 cells in cases where 1 µg/ml of β1-40 peptide was added, and the shaded graph bars indicate the same in cases where 0.1 µg/ml of β1-40 peptide was added.

Fig. 5 shows the suppressing effect of rifampicin on β1-40 cytotoxicity which was measured by the MTT assay using PC12 cells, according to Example 8.

In this figure, the shaded graph bars indicate the viability of PC12 cells in cases where no rifampicin was added, and the solid white graph bars indicate the same in cases where rifampicin was added.

Fig. 6 shows the suppressing effect of rifampicin on β1-42 peptide aggregation, according to Example 9.

### BEST MODE FOR CARRYING OUT THE INVENTION

When the rifamycins of the present invention are represented by formula [I] given above, the combinations of R₁ and R₂ in the formula may be those of, for example, a rifamycin wherein R₁ is a hydrogen atom and R₂ is a hydroxyl group or the group represented by the above formula [III] or the above formula [IV] (rifamycin SV, rifamycin B and rifamycin B diethylamide, respectively); a rifamycin wherein R₁ is the group represented by the above formula [II] and R₂ is a hydroxyl group (rifampicin); or a rifamycin wherein R₁ and R₂ together form the cyclic structure represented by the above formula [V] (rifaximin). Preferred of these combinations of R₁ and R₂ include rifamycins wherein R₁ is a hydrogen atom and R₂ is a hydroxyl group or the group represented by the above formula [III] and rifamycins wherein R₁ is a group represented by the above formula [II] and R₂ is a hydroxyl group.

The structural formulas of preferred specific cases of these rifamycins are given as (1) to (3) below.
(1) Rifampicin 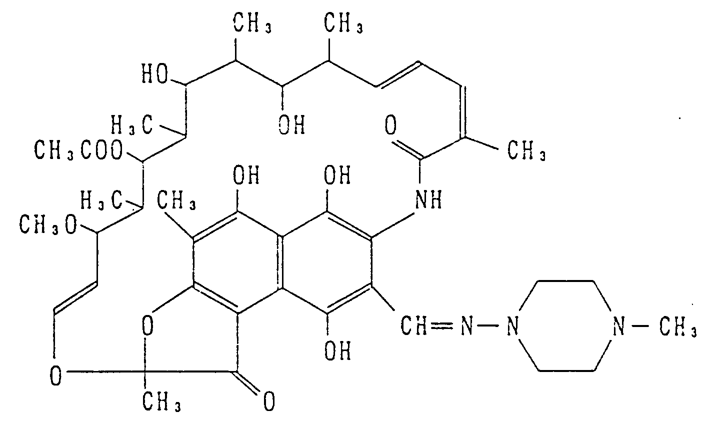
(2) Rifamycin SV 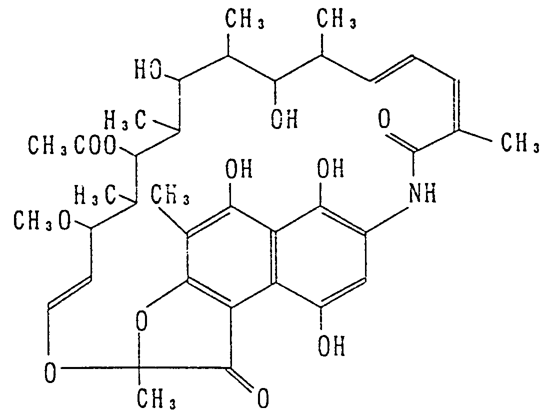
(3) Rifamycin B 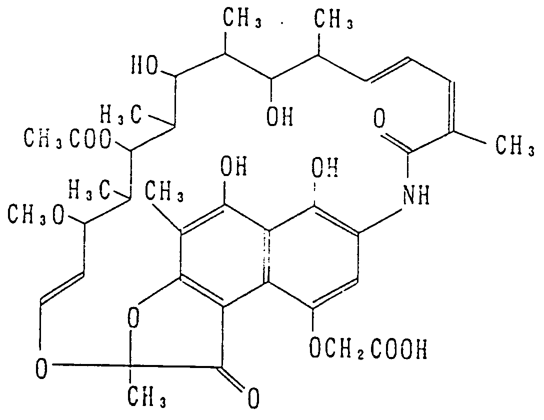
When the rifamycin of the present invention is represented by formula [VI] given above, the combinations of R₃ and R₄ may together form either a carbonyl group with the carbon atom to which they are bonded or the group represented by the above formula [VII]; specific preferred rifamycins of these types are rifamycin S and rifamycin O, respectively.

Of these, particularly preferred as the rifamycin represented by the above formula [I] of the present invention is a rifampicin wherein R₁ in formula [I] is a group represented by the above formula [II] and R₂ is a hydroxyl group.

The rifamycins of the present invention also include their salts in cases where pharmaceutically acceptable salts thereof exist which may be formed with known bases or acids (for example, inorganic and organic acids) due to acidic or basic groups being present in the molecules.

The rifamycins of the present invention are all known compounds and for example rifampicin, rifamycin SV, rifamycin B, etc. may also be obtained as reagents. Furthermore, rifamycin S may be easily synthesized by a known method (oxidation reaction using MnO₂ or H₂O₂) from rifamycin SV which is obtainable as a reagent.

The β-amyloid protein aggregation and/or deposition inhibitor of the present invention is preferably used in admixture with a pharmaceutically acceptable carrier. The pharmaceutically acceptable carrier in this case may be one of the excipients mentioned hereunder. The amount of the carrier with the compound of the present invention to be formulated depends on the dosage of the active component which is mentioned hereunder; however, unless there are particular restrictions it may be selected within a wide range, and normally the rifamycins of the present invention are used at 1 to 70 wt%, and preferably 5 to 50 wt%, of the total composition. The resulting composition may be provided in the form of soft capsules, hard capsules, tablets, granules, a powder, suspension, liquid preparation, syrup, etc. as an oral preparation, as well as injection, suppository or external preparation, by a known method using an appropriate excipient or the like.

Excipients which may be used include vegetable oils (such as corn oil, cottonseed oil, coconut oil, almond oil, peanut oil, olive oil); medium chain fatty acid glyceride oils; oily esters such as; mineral oils; glycerin esters such as tricaprilin, triacetin; alcohols such as ethanols; physiological saline; propylene glycol; polyethylene glycol; vaseline; animal fats and oils; cellulose derivatives (crystalline cellulose, hydroxypropyl cellulose, hydroxypropylmethyl cellulose, methyl cellulose); polyvinylpyrrolidone; cyclodextrin; dextrin; lactose; mannitol; sorbitol; starch; etc.

The rifamycins of the present invention and their pharmaceutically acceptable salts have inhibiting activity against β-amyloid protein aggregation and/or deposition, and thus are useful as therapeutic and/or preventive medicines against Alzheimer-type dementia.

The dosage of the activity component will depend on the severity of the condition and the age, etc. of the patient, but normally it is preferred to be about 10 to 1000 mg/day/person, and preferably 100 to 600 mg/day/person, and therefore the preparation is made to fulfill these conditions.

The rifamycins of the present invention are preferably administered as oral preparations, although their administration as injections is also possible.

The rifamycins of the present invention have low toxicity; for example, the LD₅₀ value upon oral administration of rifampicin to rats is 1720 mg/kg, and the other rifamycins have about the same low toxicity.

The test methods and measuring methods used according to the present invention are as follows.

### (1) Test method for β-amyloid protein aggregation-inhibiting activity

### (1-1) Method by sodium dodecyl sulfate-polyacrylamide gel electrophoresis (SDS-PAGE)

When synthesized β-amyloid protein is subjected to SDS-PAGE, a 3-4 kDa band due to the peptide monomer is observed. However, when the protein begins to aggregate, this monomer band becomes thinner and a smear is observed at the high molecular end (Tomiyama, et al. 1994, J. Biol. Chem. 269, 10205-10208). The inhibitory activity of a drug agent against β-amyloid protein aggregation may be determined by measuring the aggregation period of β-amyloid in admixture with the drug agent based on the decrease in peptide monomer or appearance of smearing observed with SDS-PAGE.

Specifically, 10 µl of solution is taken from a reagent solution containing β-amyloid protein and a drug agent, an equivalent of a sample buffer for electrophoresis (20 mM Tris-HCl buffer, pH 6.8, 40% glycerin, 2% SDS, 2% β-mercaptoethanol) is added thereto, and the mixture is boiled at 100°C for 5 minutes. Next, 10 µl of the solution is placed on a 15-25% polyacrylamide gradient gel for electrophoresis in a Tris-glycine buffered system. After completion of the electrophoresis, the gel is stained with 0.2% Coomassie brilliant blue solution and the amount of peptide monomer (band thickness) and presence of smearing is observed. The measurement is made periodically, and if the aggregation period of the peptide is slower compared to a solution containing only β-amyloid protein without the drug agent, the drug agent is judged to have inhibitory activity against β-amyloid protein aggregation.

### (1-2) Method using thioflavin T (ThT)

It has been reported that the pigment thioflavin T (ThT) binds to the β-sheet structure of aggregated β-amyloid protein, etc. and produces a new fluorescence (482 nm) not exhibited in its free state (Harry LeVine II. 1993, Protein Science 2, 404-410). The intensity of the fluorescence is proportional to the degree of aggregation of the protein to which it binds. By measuring the degree of aggregation of β-amyloid protein containing the drug agent based on the intensity of fluorescence of ThT binding thereto, it is possible to determine the inhibitory activity of the agent against β-amyloid protein aggregation.

Specifically, 10 µl is taken from a reagent solution of β-amyloid protein containing a certain drug agent and added to 1 ml of a ThT solution dissolved to a concentration of 3 µM in 50 mM sodium phosphate buffer solution (pH 6.0), and stirred. After stirring, the fluorescence of the solution is quickly measured using a spectrofluorometer (product of JASCO), at an excitatory wavelength of 450 nm and a fluorescent wavelength of 482 nm. The measurement is taken periodically and compared with a solution containing only β-amyloid protein without the drug agent, and if the increase in fluorescence during a given period is suppressed, the drug agent is judged to have inhibitory activity against β-amyloid protein aggregation.

### (1-3) Method by electron microscope observation

It is known that when β-amyloid protein aggregates, fibers with a diameter of about 10 nm are produced which are observable under an electron microscope (Fraser, et al. 1992, Biochemistry 31, 10716-10723, etc.). By observing drug agent-containing β-amyloid protein solutions under an electron microscope and determining the presence of such fibers, it is possible to determine the inhibitory activity of the drug agent against β-amyloid protein aggregation.

Specifically, a portion of a reagent solution containing β-amyloid protein with a certain drug agent is adsorbed onto a 200 mesh copper grid which has been coated with Formvar. After air drying, it is negatively stained with 2% uranyl acetate. The sample obtained in this manner is then enlarged to 33,000x with a transmission electron microscope and observed.

### (2) Method of measuring neurotoxicity of β-amyloid protein using PC12 cells

### (2-a) Cell preparation

PC12 cells (RCB0009, Lot 6) were used, which were provided by the Cell Bank of the Institute of Physical and Chemical Research. These cells are rat adrenal medulla-derived clones, and since upon culturing in the presence of NGF they differentiate to sympathetic ganglion cells (Greene and Tischler, Proc. Natl. Acad. Sci. USA, 73: 2424-2428, 1976), they are widely used as a model system of nerve cells.

The culturing is conducted using RPMI 1640 medium (product of Gibco) containing 10% equine serum (product of Gibco) and 5% semi-fetal bovine serum (product of Mitsubishi Kasei), at 37°C in the presence of 5% CO₂. The cells are enfolded in a 96-well culture plate (SUMILON CELLTIGHT C-1, product of Sumitomo Bakelite) at 5,000 cells/well, and on the following day the medium is exchanged with one containing 50 ng/ml NGF (rat submandibular gland-derived, product of Sigma) and no D-glucose, and at 4 days thereafter the medium is again exchanged and the culturing is continued for use in the following measurement after 7 days.

### (2-b) Measurement of cytotoxic effect of β-amyloid protein on PC12 cells by the MTT assay

After exchanging the medium (180 µl/well) of the PC12 cells on the 7th day of the above culturing (2-a), β-amyloid protein dissolved in phosphate-buffered saline (PBS) was added at 20 µl/well. After 40 hours, PBS solution containing 5 mg/ml of MTT (product of Sigma) was added at 20 µl/well, and the culturing was continued at 37°C in the presence of 5% CO₂. After 4 hours the medium was absorbed away, 0.04 N HCl/isopropanol was added at 100 µl/well, formazan was dissolved therein, and a microplate reader (Vmax, product of Molecular Devices Co.) was used for colorimetry (570-650 nm) to measure the change in MTT reduction by the PC12 cells.

### (2-c) Measurement of cytotoxic effect of β-amyloid protein on PC12 cells by the lactate dehydrogenase (LDH) assay

After exchanging the medium (180 µl/well) of the PC12 cells on the 7th day of the above culturing (2-a), β-amyloid protein dissolved in phosphate-buffered saline (PBS) was added at 20 µl/well. After 50 hours, the culture supernatant was recovered and the lactate dehydrogenase (LDH) activity of the supernatant was measured as an index of the cytotoxic activity. Here, the lactate dehydrogenase activity increases as the cell viability decreases. The measurement of the lactate dehydrogenase activity was made using a commercially available LDH assay kit (LDH-cell toxicity test kit, product of Wako Junyaku Kogyo).

The β-amyloid protein used in the test methods (1) and (2) above are β25-35 peptide, β1-40 peptide and β1-42 peptide, purchased from Bachem Co., and β1-35 peptide and β1-40 peptide obtained in Reference Example 1 below.

### EXAMPLES

The present invention will now be explained in more detail by way of the following reference examples and examples. Unless otherwise specified, the percentages in the examples, etc. are weight/volume percentages.

### Reference Example 1: Synthesis of β-amyloid protein (β1-35 peptide and β1-40 peptide)

In the examples provided hereunder, two species of β-amyloid protein were used to determine the inhibitory activity against β-amyloid protein aggregation: β1-35 and β1-40 peptides comprising 35 and 40 amino terminal residues, respectively, from the β-amyloid protein sequence of about 40-43 amino acids.

The peptide synthesis was conducted by the solid phase method with Fmoc-amino acids as the raw material, using a peptide synthesizer (product of Applied Biosystems Co.). After completion of the synthesis, the peptides were cut out of the resin and purified by reverse phase high performance liquid chromatography using a C₁₈ column (product of Waters Co.). The obtained β1-35 peptide and β1-40 peptide were confirmed to include the object amino acid sequence by use of a peptide sequencer (product of Applied Biosystems Co.). The β1-35 peptide and β1-40 peptide obtained in this manner were lyophilized and used for the following examples.

### Example 1

The β1-35 peptide obtained in Reference Example 1 was dissolved in double deionized water to a final concentration of 4 mg/ml and passed through a 0.22 µm filter, after which it was mixed at a ratio of 1:1 with a 0.2 M sodium phosphate buffer (pH 7.4) containing 0.02% sodium azide (antiseptic) to obtain a β1-35 peptide solution with a final concentration of 2 mg/ml. This β1-35 peptide solution was dispensed into 5 Eppendorf tubes (200 µl/tube). Rifampicin, rifamycin SV and rifamycin B (all products of Sigma Co.) dissolved in 100% methanol to a concentration of 10 mg/ml were added at 1% by volume to each of 3 tubes (final concentration of drug agents: 100 µg/ml), 100% methanol alone was added to another tube at 1%, and the remaining tube was used as a control with no addition.

The tubes holding the β1-35 peptide solutions containing rifampicin, rifamycin SV or rifamycin B, the methanol-containing β1-35 peptide solution and the control β1-35 peptide solution were allowed to stand at room temperature. A portion of each of the solutions was taken periodically and subjected to SDS-PAGE according to the test method described previously ((1-1) Method by SDS-PAGE), and the inhibitory activities of each of the drug agents against β1-35 peptide aggregation were compared. As a result, in the control β1-35 peptide solution and the methanol-containing β1-35 peptide solution there were observed reductions in peptide monomer and appearance of smear by the 7th day after dissolution, while in the β1-35 peptide solutions containing rifampicin, rifamycin SV or rifamycin B, no reduction in peptide monomer or smearing was observed up to the 14th day after dissolution.

These results indicate that rifampicin, rifamycin SV and rifamycin B at a concentration of 100 µg/ml suppress aggregation of 2 mg/ml of β-amyloid protein (β1-35 peptide).

### Example 2

Following the control method of Example 1, β1-35 peptide at a concentration of 1 mg/ml was allowed to stand for about 1 month to form a smear. This was added at 1% by volume to a just-solubilized 1 mg/ml β1-35 solution, and then the obtained β1-35 peptide solution was dispensed into 4 vials at 2 ml each (1 mg/ml). To one of the vials was added 10 mg/ml of rifampicin dissolved in methanol, to an amount of 0.1% (final concentration of rifampicin: 10 µg/ml), to another vial was added 1 mg/ml of rifampicin also dissolved in methanol, also to an amount of 0.1% (final concentration of rifampicin: 1 µg/ml), to yet another vial was added methanol alone to 0.1%, and the last vial was used as a control with no addition. The vials containing the 10 µg/ml rifampicin-containing β1-35 peptide solution, the 1 µg/ml rifampicin-containing β1-35 peptide solution, the methanol-containing β1-35 peptide solution and the control β1-35 peptide solution were allowed to stand at room temperature and were subjected to SDS-PAGE periodically in the same manner as in Example 1, and the appearance periods of β1-35 peptide aggregates (smears) were measured according to the test method described previously.

As a result, in the control, methanol-containing and 1 µg/ml rifampicin-containing β1-35 peptide solutions, a smear was observed on the 6th day after dissolution, whereas in the 10 µg/ml rifampicin-added solution, no smear was observed up to the 10th day after dissolution. These results indicate that the commonly used concentration of rifampicin is effective for suppression of β-amyloid protein aggregation after nucleation.

### Example 3

The effect of rifampicin on β25-35 peptide cytotoxicity on PC12 cells was determined by the test method described above ((2-b) Measurement of cytotoxic effect of β-amyloid protein on PC12 cells by the MTT assay).

That is, at the time of dissolving the β25-35 peptide in PBS, a rifampicin (product of Sigma)-containing dimethylsulfoxide (DMSO) solution was added in an amount of 1/100 and stirred, and the mixture was added to PC12 cells.

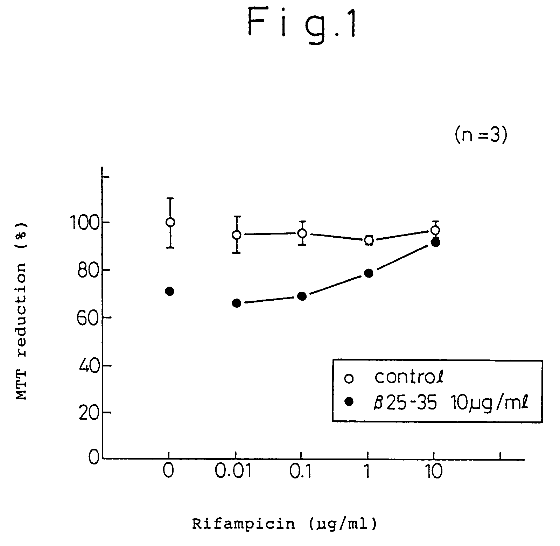

As shown in Fig. 1, in the case of 0 µg/ml rifampicin in the presence of 10 µg/ml of β25-35 peptide, the MTT reduction-lowering effect of β25-35 on PC12 cells was observed, whereas rifampicin suppressed the MTT reduction-lowering effect of β25-35 on PC12 cells in a dose-dependent manner. These results indicate that rifampicin has an effect of suppressing the neurotoxicity of β-amyloid protein.

### Example 4

The 4 rifamycin derivatives rifampicin (A), rifamycin B (B), rifamycin SV (C) and rifamycin S (D) were each dissolved in dimethylsulfoxide (DMSO) at 10 mg/ml.

The β1-35 peptide obtained in Reference Example 1 was dissolved in double deionized water to 2 mg/ml and passed through a 0.22 µm filter, after which it was mixed at a ratio of 1:1 with a 0.2 M sodium phosphate buffer (pH 7.4) to obtain a β1-35 peptide solution with a final concentration of 1 mg/ml. This solution was dispensed into 15 Eppendorf tubes at 200 µl each.

One of the 10 mg/ml rifamycin derivative solutions prepared above was added at 2 µl each to 3 of the tubes containing the β1-35 peptide solution (final concentration of rifamycin derivative: 100 µg/ml). The other 3 rifamycin derivatives were also added at 2 µl each to 3 tubes in the same manner (A-D).

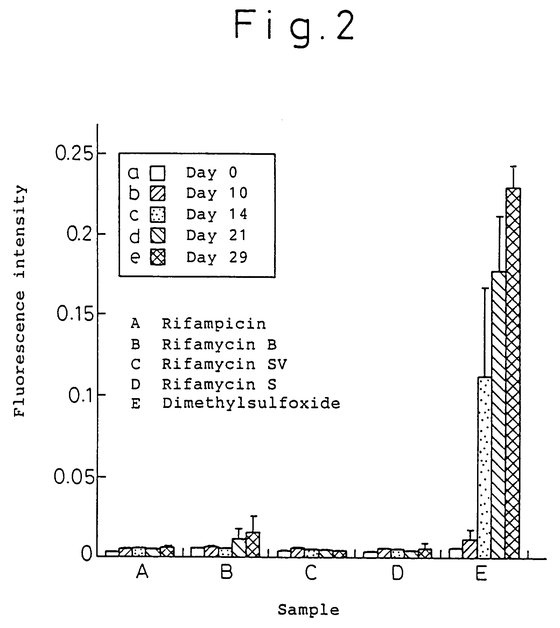

To each of the last 3 tubes, 2 µl of DMSO was added, and this was used as the control (E). The rifamycin derivative-containing β1-35 peptide solutions (A-D) and the DMSO-containing β1-35 peptide solution (E) were allowed to stand at room temperature. A portion of each of the solutions was taken periodically according to the test described above ((1-2) Method using ThT) to measure the degree of β1-35 peptide aggregation. As a result, as shown in Fig. 2, with the DMSO-containing β1-35 peptide solution (E) there was an increase in fluorescence by the 14th day after peptide dissolution, i.e. peptide aggregation occurred, and the fluorescence further increased by the 21st and 29th days. In contrast, with the rifamycin derivative-containing β1-35 peptide solutions (A-D), no increase in fluorescence was seen up to the 29th day in any of the cases.

The results described above indicate that the rifamycin derivatives of the present invention have an effect of suppressing aggregation of β-amyloid protein even with the ThT method.

### Example 5

Rifampicin was dissolved in DMSO to 10, 1 and 0.1 mg/ml. β1-40 peptide (Bachem Co.) was dissolved in double deionized water to 200 µg/ml, the insoluble fraction was removed by centrifugation at 10,000 x g for 5 minutes, and it was then passed through a membrane filter with a pore size of 0.22 µm, after which it was mixed at a ratio of 1:1 with a 0.2 M sodium phosphate buffer (pH 7.4) to obtain a β1-40 peptide solution with a final concentration of 100 µg/ml. This solution was distributed into 15 Eppendorf tubes at 200 µl each. The 10 mg/ml rifampicin solution prepared above was added at 2 µl to 3 tubes containing the β1-40 peptide solution (final rifampicin concentration: 100 µg/ml; (C)). The rifampicin solutions at the other concentrations were also each added at 2 µl to 3 tubes in the same manner (final rifampicin concentrations: 10 and 1 µg/ml; (D), (E)). Of the remaining 6 tubes, there was added 2 µl of DMSO (B) to 3 tubes, while nothing was added to the other 3 tubes which were used as the control (A).

The rifampicin-containing β1-40 peptide solutions ((C)-(E)), the DMSO-containing β1-40 peptide solution (B) and the control β1-40 peptide solution (A) were allowed to stand at room temperature. A portion of each of the solutions was taken periodically according to the test described above ((1-2) Method using ThT) to measure the degree of β1-40 peptide aggregation.

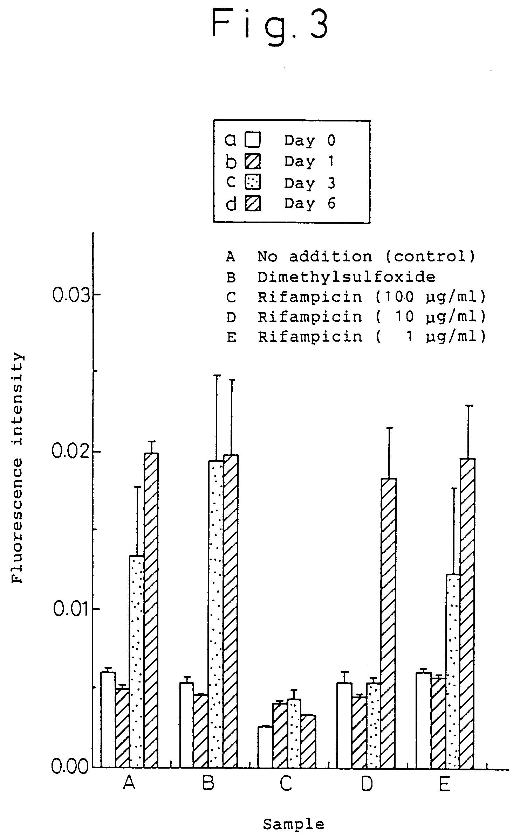

As a result, as shown in Fig. 3, with the control β1-40 peptide solution (A) and the DMSO-containing β1-40 peptide solution (B) there was an increase in fluorescence by the 3rd day after the dissolution of peptide, i.e. peptide aggregation occurred, and the fluorescence further increased by the 6th day. In contrast, with the β1-40 peptide solution containing 100 µg/ml of rifampicin (C), no increase in fluorescence was seen up to the 6th day. With the β1-40 peptide solution containing 10 µg/ml of rifampicin (D), no fluorescence was seen up to the 3rd day, but by the 6th day the same degree of fluorescence was exhibited as with the control peptide solution. On the other hand, the peptide solution containing 1 µg/ml of rifampicin (E) followed the same course as the control peptide solution. These results indicate that rifampicin suppresses aggregation of β-amyloid protein in a concentration-dependent manner and that 100 µg/ml of rifampicin completely suppresses aggregation of 100 µg/ml of β1-40 peptide.

β-amyloid protein is contained in normal human cerebrospinal fluid at a concentration of 2.5 ng/ml (Seubert, et al. Nature 359, 325-327, 1992). On the other hand, rifampicin is normally administered orally to patients at about 300-600 mg/day, to a maximum blood concentration of about 10 µg/ml. It has also been reported that about 10% of the rifampicin blood concentration migrates to the brain (Mindermann, et al. J. Antimicrobial Chemotherapy 31, 731-737, 1993). That is, the rifampicin concentration in the brain is usually expected to be a maximum of 1 µg/ml. Consequently, the results obtained in Example 5 indicate that the normally administered dose of rifampicin is capable of adequately suppressing aggregation of β-amyloid protein in the brain.

### Example 6

Following the method of Example 5, a β1-40 peptide solution containing 100 µg/ml of rifampicin and a β1-40 peptide solution containing 1% DMSO were prepared (peptide concentration: 100 µg/ml in both cases) and allowed to stand at room temperature. A portion of each solution was taken on the 8th day, and fibril formation due to peptide aggregation was observed according to the test method described above ((1-3) Method by electron microscope observation).

As a result, with the DMSO-containing β1-40 peptide solution, a large number of fibrils 5-20 nm in diameter were observed, whereas with the rifampicin-containing β1-40 peptide solution virtually none were observed. These results indicate that rifampicin has an effect of suppressing fibril formation due to aggregation of β-amyloid protein.

### Example 7

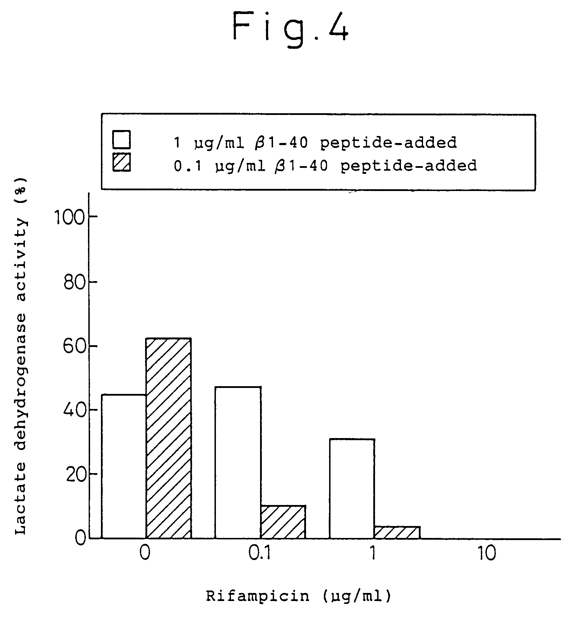

The effect of rifampicin on β1-40 peptide cytotoxicity on PC12 cells viability was investigated by the test method described above ((2-c) Measurement of cytotoxic effect of β-amyloid protein on PC12 cells by the lactate dehydrogenase (LDH) assay), and the results are shown in Fig. 4. That is, β1-40 peptide was dissolved in double deionized water to 200 µg/ml, the insoluble fraction was removed by centrifugation at 10,000 x g for 5 minutes, and it was then passed through a membrane filter with a pore size of 0.22 µm, after which it was mixed at a ratio of 1:1 with a 0.2 M sodium phosphate buffer (pH 7.4) to obtain a phosphate-buffered β1-40 peptide solution with a final concentration of 100 µg/ml. This solution was allowed to stand for 7 days at room temperature, after which a dimethylsulfoxide (DMSO) solution containing rifampicin (product of Sigma Co.) was mixed therewith, and added to PC12 cell culture to final concentrations of β1-40 peptide of 1 µg/ml and 0.1 µg/ml.

As shown in Fig. 4, in the case of addition of β1-40 peptide in the absence of rifampicin, the lactate dehydrogenase activity in the cell culture supernatant increased, whereas this increase in lactate dehydrogenase activity due to β1-40 peptide was suppressed by rifampicin in a dose-dependent manner, and was completely suppressed with 10 µg/ml. These results indicate that rifampicin has an effect of suppressing the neurotoxicity of β-amyloid protein.

### Example 8

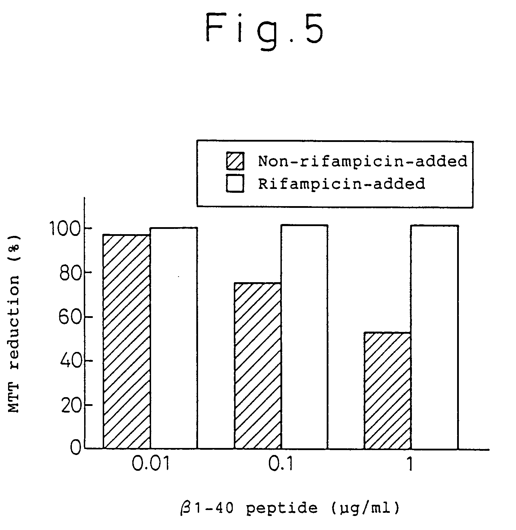

The suppressing effect of rifampicin against neurotoxicity due to β-amyloid protein was investigated using the β1-40 obtained in Reference Example 1, by the test method described above ((2-b) Measurement of cytotoxic effect of β-amyloid protein on PC12 cells by the MTT assay), and the results are shown in Fig. 5. That is, β1-40 peptide was dissolved in double deionized water and the insoluble fraction was removed by centrifugation at 10,000 x g for 5 minutes, after which a peptide concentration of 90 µg/ml was prepared and mixed at a ratio of 1:1 with a 0.2 M sodium phosphate buffer (pH 7.4) to obtain a phosphate-buffered β1-40 peptide solution with a final concentration of 45 µg/ml. To this solution was added a DMSO solution containing rifampicin at 1 mg/ml, to prepare a final rifampicin concentration of 10 µg/ml, and the mixture was then incubated at 37°C for 7 days. Also, as a control, a phosphate-buffered β1-40 peptide solution containing no rifampicin was also incubated at 37°C for 7 days. After 7 days of incubation, the solutions were added to PC12 cells to final β1-40 peptide concentrations of 1 µg/ml, 0.1 µg/ml and 0.01 µg/ml.

As shown in Fig. 5, in the case where the β1-40 peptide incubated without addition of rifampicin was added to the cells, a concentration-dependent cytotoxicity was found for the β1-40 peptide, whereas in the case where the β1-40 peptide incubated after addition of rifampicin was added to the cells, no cytotoxicity was observed at any of the β1-40 peptide concentrations. These results indicate that rifampicin has an effect of suppressing the neurotoxicity of β-amyloid protein.

### Example 9

A 100 µg portion of β1-42 peptide (product of Bachem Co.) was dissolved in 200 µl of DMSO, and the solution was further diluted with double deionized water to 5.5-fold to obtain a 20 µM β1-42 solution. The solution was distributed into 12 Eppendorf tubes at 50 µl each. Next, 1 µl of rifampicin solutions dissolved in DMSO to concentrations of 10 mM, 1 mM and 0.1 mM were each added to three of the above-mentioned tubes, and as a control 1 µl of DMSO alone was added to three of the tubes. To each of these was added 50 µl of 2-fold concentration of PBS and the mixtures were stirred, to obtain 10 µM β1-42 solutions (10% DMSO-containing PBS solutions) either containing rifampicin at final concentrations of 100 µM, 10 µM or 1 µM, or containing none. These were incubated at 37°C, and one week later the aggregation of the β1-42 peptide was measured according to the test method described previously ((1-2) Method using ThT).

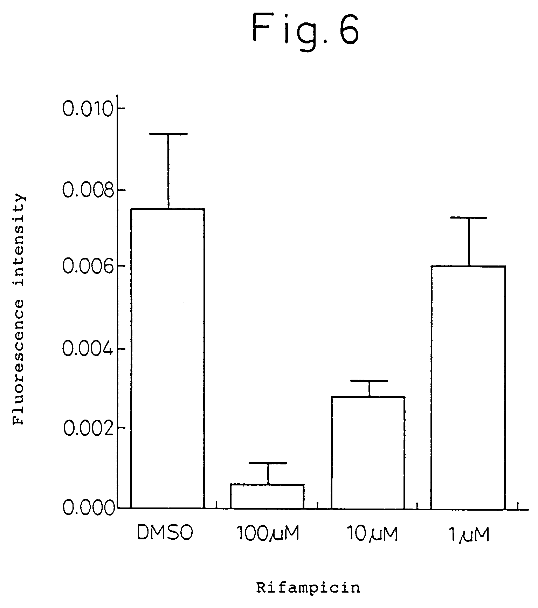

As a result, as shown in Fig. 6, rifampicin suppressed the β1-42 peptide aggregation in a concentration-dependent manner. These results indicate that rifampicin is also effective at a reasonable concentration against aggregation of β1-42 peptide, which, of the various lengths of β-protein, is implicated as the principal cause of amyloid deposition.

### Example 10

### Preparation of capsules

Capsules containing 100 mg of rifampicin were prepared with the following composition per capsule.

| | |
|---|---|
| Rifampicin | 100.00 mg |
| Lactose | 50.00 g |
| Ethyl cellulose | 1.50 g |
| Stearic acid | 1.50 g |

The rifampicin and lactose were combined and then moistened with a methylene chloride solution containing ethyl cellulose, and finally fine granules were obtained. Finely crushed stearic acid was mixed therewith to obtain a powder. The powder obtained in this manner was used to fill capsules (#2) to obtain an agent in capsule form.

### Industrial Applicability

Drug agents containing rifamycins represented by the above formula [I] or [VI] of the present invention as the active components have an effect of suppressing aggregation of β-amyloid protein, as well as an effect of suppressing neurotoxicity due to β-amyloid protein, and are thus expected to be useful as therapeutic and/or preventive medicines for Alzheimer-type dementia.

## Claims

1. A β-amyloid protein aggregation and/or deposition inhibitor which contains as an active component a rifamycin represented by the following formula [I] 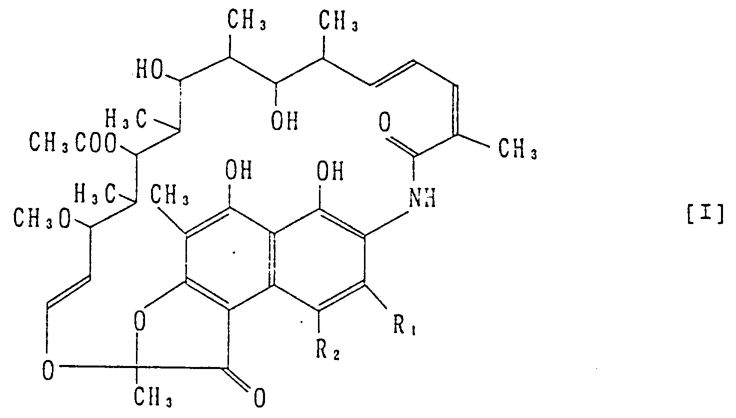 wherein R₁ is a hydrogen atom or the group represented by the following formula [II] 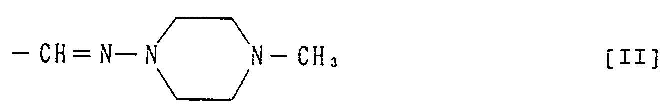 and R₂ is a hydroxyl group or the group represented by the following formula [III] or [IV]
-OCH₂COOH [III]
-OCH₂CON(C₂H₅)₂ [IV]
or R₁ and R₂ may together form the cyclic structure represented by the following formula [V] 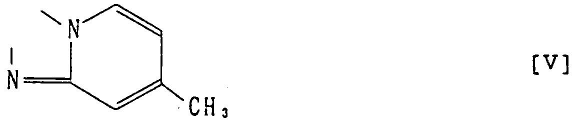 or by the following formula [VI] 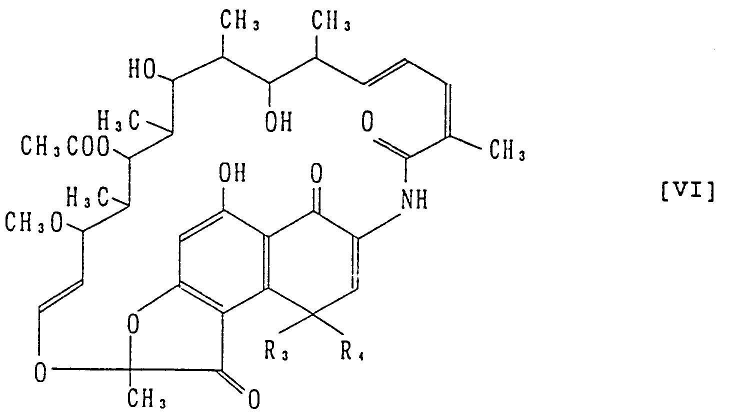 wherein R₃ and R₄ together form a carbonyl group with the carbon atom to which they are bonded or the group represented by the following formula [VII] 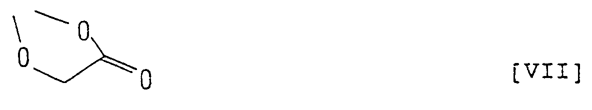 or a pharmaceutically acceptable salt thereof.

2. A β-amyloid protein aggregation and/or deposition inhibitor according to Claim 1, wherein in said formula [I], R₁ is a hydrogen atom and R₂ is a hydroxyl group or the group represented by said formula [III], or R₁ is a group represented by said formula [II] and R₂ is a hydroxyl group.

3. A β-amyloid protein aggregation and/or deposition inhibitor according to Claim 1, wherein in said formula [I], R₁ is a group represented by said formula [II] and R₂ is a hydroxyl group.

4. A therapeutic or preventive medicine for Alzheimer-type dementia which contains, as the active component rifamycin, a rifamycin represented by said formula [I] or said formula [VI], or a pharmaceutically acceptable salt thereof.

5. A therapeutic or preventive medicine for Alzheimer-type dementia according to Claim 4, wherein the active component rifamycin is a rifamycin represented by said formula [I] or a pharmaceutically acceptable salt thereof, and in said formula [I], R₁ is a hydrogen atom and R₂ is a hydroxyl group or the group represented by said formula [III], or R₁ is a group represented by said formula [II] and R₂ is a hydroxyl group.

6. A therapeutic or preventive medicine for Alzheimer-type dementia according to Claim 4, wherein the active component rifamycin is a rifamycin represented by said formula [I] or a pharmaceutically acceptable salt thereof, and in said formula [I], R₁ is a group represented by said formula [II] and R₂ is a hydroxyl group.
